# FASCICULE DE BREVET EUROPEEN

(11) **EP 0 318 394 B1**
(45) Date de publication et mention de la délivrance du brevet: **02.02.1994**
(21) Numéro de dépôt: 88402975.2
(22) Date de dépôt: 25.11.1988
(51) Int. Cl.: C07C 323/20, C07C 319/06

(54) **Procédé d'alkylthiolation des phénols et son application à la synthèse d'acyl-4 alkylthio-2 phénols**
Verfahren zur Thioalkylierung von Phenolen und seine Anwendung in der Synthese von 4-Acyl-2-alkylthiophenolen
Process for the alkylthiolation of phenols and its application in the synthesis of 4-acyl-2-alkylthiophenols

(30) Priorité: 27.11.1987 FR 8716508; 27.11.1987 FR 8716509
(43) Date de publication de la demande: 31.05.1989
(73) Titulaire: SOCIETE NATIONALE ELF AQUITAINE (PRODUCTION), 92400 Courbevoie (FR)
(72) Inventeur: Vottero, Catherine, F-77160 Provins (FR); Labat, Yves, F-64000 Pau (FR); Poirier, Jean-Marie, F-76160 Saint Martin Du Vivier (FR)
(74) Mandataire: Leboulenger, Jean

(56) Documents cités:
- DE-A- 2 728 641
- FR-A- 2 398 753
- US-A- 2 923 743
- US-A- 4 324 920
- HOUBEN-WEYL - METHODEN DER ORGANISCHEN CHEMIE, tome VII/2A, partie 1, 1973, Georg Thieme Verlag, Stuttgart, DE; C.-W. SCHELLHAMMER: "Ketone aus Carbonsäuren und Aromaten bzw. reaktionsfähigen Heteroaromaten in der Gegenwart von Bortrifluorid"

## Description

La présente invention concerne la préparation d'alkylthio-phénols par réaction d'un disulfure de dialkyle avec un phénol et a plus particulièrement pour objet la préparation d'alkylthio-2 phénols et leur transformation en acyl-4 alkylthio-2 phénols.

Les alkylthio-phénols sont des produits connus, utilisés notamment comme intermédiaires en chimie pharmaceutique pour la préparation de médicaments hypotenseurs et vasodilateurs ou en agrochimie pour celle d'herbicides ou de pesticides. Pour ces utilisations, il est particulièrement important de disposer spécifiquement de l'isomère ortho ou de l'isomère para.

Pour préparer sélectivement chaque isomère, les méthods connues font généralement intervenir un produit de départ correctement disubstitué (chloronitrobenzène, nitrophénol, mercaptophénol, dichlorobenzène) ; ces méthodes comprennent de nombreuses étapes et les rendements obtenus sont souvent très faibles.

Parmi les nombreuses voies d'accès aux alkylthiophénols, la plus simple consiste à faire réagir un disulfure de dialkyle avec un phénol en présence d'un acide de Lewis. Cependant, les procédés décrits à ce jour ne permettent pas d'associer une bonne sélectivité et un taux élevé de conversion du phénol de départ. Ainsi, par example, par réaction du phénol et du disulfure de diméthyle (DMDS) en présence de chlorure ferrique ou de chlorure d'aluminium dans le chlorobenzène (brevet US 2 923 743), la sélectivité isomère ortho/isomère para n'est que de 85/15 et le rendement ne dépasse pas 48 %. Selon le même brevet, en l'absence de solvant et avec une terre décolorante (Tonsil), l'isomère para est prépondérant, mais le rendement maximal est de 37 %.

La réaction du phénol et du DMDS a également été étudiée par Farah et Gilbert (brevet BE 627 306 et J. Org. Chem. 28, 2807, 1963). En utilisant comme acide de Lewis soit un acide sulfonique, soit le système P₂O₅/H₃PO₄, soit encore une résine acide (polymère sulfoné Dowex-50), ces auteurs ont obtenu des taux de conversion de l'ordre de 20 à 30 % avec une sélectivité para/ortho de 82/18.

Plus récemment (Synthesis 117, 1984 et brevet US 4 324 920), Ranken et McKinnie ont proposé d'effectuer la réaction d'un phénol et d'un disulfure de dialkyle en l'absence de solvant en utilisant comme catalyseur un phénate d'aluminium. Dans le cas du phénol et du DMDS, cette méthode conduit à une sélectivité ortho/para de 71/29 et les rendements en méthylthio-2 phénol sont de l'ordre de 40 % seulement.

D'autre part, les acyl-4 alkylthio-2 phénols qui sont utiles comme intermédiaires en chimie pharmaceutique pour la synthèse de médicaments hypotenseurs et vasodilatateurs (voir par exemple le brevet GB 1 544 872 et les brevets US 4 124 722, 4 327 224 et 4 374 149) peuvent être obtenus de deux façons. La première qui consiste à sulfochlorer un acyl-4 phénol, puis à réduire le sulfochlorure obtenu en acyl-4 mercapto-2 phénol et à traiter ce dernier par un agent d'alkylation, conduit à de faibles rendements ; ainsi, à partir de l'acétyl-4 phénol, le rendement global en acétyl-4 méthylthio-2 phénol (ou hydroxy-4 méthylthio-3 acétophénone) n'est que de 22 %. La seconde méthode qui consiste à acyler directement un alkylthio-2 phénol au moyen d'un halogénure d'acide sous des conditions de Friedel-Crafts (AlCl₃ dans nitrobezène) fournit de meilleurs rendements, mais cependant encore très faibles (34 % pour l'acétylation du méthylthio-2 phénol en hydroxy-4 méthylthio-3 acétophénone).

L'acylation du phénol au moyen d'un acide carboxylique en présence de trifluorure de bore a été décrite pour la première fois en 1933 par H. Meervein (Ber. dtsch. Chem. Ges. 66, 411). Bien que cette méthode ait fait depuis l'objet de nombreux travaux parmi lesquels on peut citer plus particulièrement ceux de N.P. Buu-Hoï et al. (J. Org. Chem. 20, 606, 1955), E.C. Armstrong et al. (J. Am. Chem. Soc. 82, 1928, 1960), K. Freundenberg et al. (Ann. Chem. 590, 140, 1954) et K. Kindler et al. (Archiv der Pharmazie 287, 210, 1954), elle n'a encore jamais été appliquée aux alkylthio-2 phénols. D'autre part, dans les conditions habituellement préconisées par la littérature (1 à 2 moles de complexe BF₃/acide par mole de phénol), l'application de cette méthode aux alkylthio-2 phénols ne conduit qu'à de très faibles rendements (inférieurs à 40 %).

Il a maintenant été trouvé que, sous certaines conditions opératoires, la réaction d'un phénol avec un disulfure de dialkyle permet d'obtenir les alkylthio-2 phénols avec une sélectivité et un rendement excellents.

Il a également été trouvé que les alkylthio-2 phénols peuvent être acylés par un complexe BF₃/acide avec d'excellents rendements à condition d'opérer dans une certaine zone de température et d'utiliser une proportion élevée de complexe BF₃/acide.

Le procédé d'alkylthiolation selon l'invention qui consiste à faire réagir un phénol comportant au moins un atome d'hydrogène en ortho du groupe hydroxyle et un disulfure de dialkyle linéaire, est caractérisé en ce qu'on utilise comme acide de Lewis le chlorure d'aluminium et qu'on opère au sein d'un solvant du type alkyl-benzène choisi parmi le toluène, l'éthylbenzène et le cumène.

Le procédé selon l'invention est plus particulièrement destiné à l'alkylthiolation du phénol, mais il peut également être appliqué à des phénols porteurs d'un ou plusieurs groupes activants (alkyl en C₁ à C₉, phényl, hydroxy) ou modérément attracteurs (par exemple Cl) tels que le paracrésol, le diméthyl-2,4 phénol, le tert-butyl-4 phénol, le di(tert-butyl)-2,4 phénol, l'hydroquinone et le parachlorophénol. Lorsqu'on utilise le chlorure d' aluminium comme acide de Lewis, l'alkylthiolation s'effectue essentiellement en ortho du groupe hydroxyle avec une sélectivité ortho/para pouvant atteindre et même parfois dépasser 95/5.

Les disulfures de dialkyle linéaires conformes à l'invention peuvent contenir jusqu'à 18 atomes de carbone. On préfère cependant utiliser le disulfure de diméthyle (DMDS).

La quantité de disulfure de dialkyle à utiliser par mole de phénol peut varier de 1 à 10 moles, mais est de préférence comprise entre 1 et 5 moles.

Le solvant alkyl-benzène préféré est le toluène, mais on peut utiliser également l'éthylbenzène et le cumène.

La quantité de solvant alkyl-benzène peut varier dans de larges limites, mais est généralement comprise entre 0,2 et 4 litres par mole de phénol, de préférence entre 0,5 et 2 litres.

Le chlorure d'aluminium doit être utilisé en une quantité qui est au moins égale à la stoechiométrie et peut aller jusqu'à 10 moles par mole de phénol. On obtient les meilleurs résultats en utilisant le chlorure d' aluminium en une quantité allant de 1 à 3 moles par mole de phénol.

La réaction qu'il n'est pas nécessaire de réaliser sous atmosphère inerte, peut être menée à une température allant d'environ 0°C jusqu'au reflux. En général, on obtient les meilleurs résultats en travaillant à une température allant de 25 à 120°C.

Selon la présente invention, on obtient les acyl-4 alkylthio-2 phénols avec un rendement de l'ordre de 80 % et même plus en faisant réagir à une température allant de 40 à 100°C un alkylthio-2 phénol et un complexe BF₃ : 2 RCOOH dans lequel R représente un radical alkyle linéaire contenant de 1 à 12 atomes de carbone ou un radical propène-1 yle, à raison de 10 à 15 moles de complexe par mole de phénol.

Le complexe BF₃ : 2 CH₃COOH est un produit commercial. Les autres peuvent être préparés par simple barbotage de trifluorure de bore gazeux dans l'acide RCOOH correspondant, soit à la température ambiante pour les acides liquides, soit au point de fusion de l'acide pour les composés normalement solides.

La réaction d'acylation selon l'invention s'effectue en l'absence de solvant à une température allant de 40 à 100°C, de préférence entre environ 60 et 80°C. On obtient les meilleurs rendements en utilisant environ 12 moles de complexe BF₃ : 2 RCOOH par mole d'alkylthio-2 phénol.

La réaction est en général très rapide (de l'ordre de 2 à 5 heures) et son évolution peut être suivie par chromatographie en phase gaz. Quand elle est terminée, l'excès de complexe BF₃ : 2 RCOOH peut être détruit par un traitement aqueux. Il est cependant plus avantageux de déplacer le complexe BF₃ : 2 RCOOH en excès par traitement à l'éther ; l'éthérate de trifluorure de bore et l'acide RCOOH formés sont ensuite piégés à une température d'environ 30°C sous pression réduite (environ 67 Pa) de façon à éviter une dégradation observée à des températures plus élevées. Cette manière de procéder permet de récupérer l'excès de trifluorure de bore.

Les exemples suivant illustrent l'invention sans la limiter.

### EXEMPLE 1

A une solution de 0,94 g (10 mmoles) de phénol dans 15 ml de toluène, on ajoute 1,6 g (12 mmoles) de chlorure d'aluminium, puis 2,66 ml (30 mmoles) de DMDS. Le mélange, fortement agité, est ensuite porté à 105°C et maintenu pendant 14 heures à cette température. Après refroidissement à une température inférieure à 40°C, on hydrolyse par 10 ml d'une solution d'acide chlorhydrique à 10 % (en volume). On extrait ensuite avec du dichlorométhane (5 fois 16 ml), puis on évapore les solvants et purifie le méthylthio-2 phénol par chromatographie éclair sur 30 g de silice en utilisant comme éluant un mélange de 100 parties en volume d'éther de pétrole (point d'ébullition : 40-60°C) et 6 parties en volume d'éther diéthylique.

La purification peut également être réalisée sur Florisil (silicate de magnésium - cf. Merck Index 9ème édition n° 5514 en utilisant comme éluant un mélange d'éther de pétrole et d'éther diéthylique dans le rapport volumique 100/1.

Le rendement en méthylthio-2 phénol est de 94 %. Outre le méthylthio-2 phénol, on récupère environ 4 % de di(méthylthio)-2,4 phénol et 2 % de phénol. Il n'y a pas de méthylthio-4 phénol.

En opérant de la même façon, mais avec une durée de réaction de seulement 8 heures, le rendement en méthylthio-2 phénol est de 92 %.

### EXEMPLE 2

A une solution de 0,94 g de phénol dans 20 ml de toluène, on ajoute 1,33 g (10 mmoles) de chlorure d'aluminium, puis 3,69 ml (30 mmoles) de disulfure de diéthyle. La suspension, fortement agitée, est ensuite portée à 90°C et maintenue pendant 6 heures à cette température.

Le mélange réactionnel est ensuite traité de la même façon qu'à l'exemple 1 et purifié sur Florisil avec l'éther de pétrole comme éluant. On obtient ainsi l'éthylthio-2 phénol avec un rendement de 82 %.

### EXEMPLE 3

On répète l'exemple 2, mais en remplaçant le disulfure de diéthyle par 5,64 ml de disulfure de dibutyle et en maintenant à 100°C pendant 5 heures. La purification est effectuée sur Florisil en utilisant comme éluant un mélange éther de pétrole/éther diéthylique (rapport volumique 100/3). On obtient ainsi le butylthio-2 phénol avec un rendement de 79 %.

### EXEMPLE 4

A une solution de 1,08 g (10 mmoles) de paracrésol dans 20 ml de toluène, on ajoute 1,6 g de chlorure d'aluminium, puis 2,66 ml de DMDS. La suspension, vigoureusement agitée, est ensuite portée à 100°C et maintenue pendant 5 heures à cette température.

Après traitement du mélange réactionnel de la même façon qu'à l'exemple 1 et purification sur Florisil avec, comme éluant, un mélange éther de pétrole/éther diéthylique (rapport volumique 100/0,5), on obtient le méthyl-4 méthylthio-2 phénol avec un rendement de 92 %.

### EXEMPLE 5

A une solution de 1,5 g (10 mmoles) de para-tert.butyl-phénol dans 20 ml de toluène, on ajoute 1,33 g de chlorure d'aluminium, puis 1,6 ml de DMDS. La suspension, vivement agitée, est ensuite portée et maintenue à 100°C pendant 5 heures.

Après traitement et purification comme à l'exemple 1, on obtient le méthylthio-2 phénol avec un rendement de 70 %. Le groupe tert-butyle a été éliminé.

### EXEMPLE 6

Dans un réacteur de 5 litres, on introduit 203,6 g de phénol et 3,25 litres de toluène, puis on porte au reflux pour sécher éventuellement les réactifs. On ajoute ensuite 346 g de chlorure d' aluminium anhydre et 610 g de DMDS, puis on maintient la température à 105°C pendant 14 heures. Après refroidissement, on ajoute 1,1 litre d'acide chlorhydrique à 20 %, puis on décante la phase organique et la phase aqueuse est extraite par du dichlorométhane.

Après séparation des solvants et du DMDS en excès par distillation sous vide, on obtient 394 g d'un produit organique qu'on purifie par distillation. On recueille finalement 275 g de méthylthio-2 phénol ce qui correspond à un rendement de 91 % par rapport au phénol de départ.

### EXEMPLE 7

A 5 mmoles de méthylthio-2 phénol (0,7 g), on ajoute 60 mmoles de complexe BF₃ : 2 CH₃COOH (11,3 g), puis on porte la solution à 70°C et la maintient pendant 3 heures à cette température. Elle est ensuite hydrolysée par 5 ml d'une solution d'acide chlorhydrique à 10 % (en volume) et l'hydroxy-4 méthylthio-3 acétophénone est purifiée par chromatographie éclair sur 15 g de silice en utilisant comme éluant un mélange de volumes égaux d'éther de pétrole (Eb : 40-60°C) et d'éther diéthylique.

Le rendement en hydroxy-4 méthylthio-3 acétophénone est de 87 %.

### EXEMPLE 8

Une solution de 0,7 g de méthylthio-2 phénol dans 11,3 g de complexe BF₃ : 2 CH₃COOH est portée à 70°C et maintenue pendant 3 heures à cette température. Après retour à la température ambiante, on ajoute 11 ml d'éther diéthylique et on agite fortement pendant environ 5 minutes avant de distiller à pression atmosphérique l'éther en excès. L'acide acétique et l'éthérate de trifluorure de bore formés sont ensuite piégés sous 67 Pa à environ 30°C.

L'hydroxy-4 méthylthio-3 acétophénone est purifiée comme à l'exemple 13. Rendement : 86 %.

### EXEMPLE 9

On opère comme à l'exemple 7, mais en remplaçant le complexe BF₃ : 2 CH₃COOH par 12,9 g du complexe BF₃ : 2 C₂H₅COOH. On obtient ainsi avec un rendement de 84 % l'hydroxy-4 méthylthio-3 propiophénone.

### EXEMPLE 10

On répète l'exemple 7, mais en remplaçant De méthylthio-2 phénol par 0,77 g d'éthylthio-2 phénol et en utilisant comme éluant un mélange de 2 volumes d'éther de pétrole (Eb : 40-60°C) et de 1 volume d'éther diéthylique. On obtient ainsi l'éthylthio-3 hydroxy-4 acétophénone avec un rendement de 77 %.

De la même façon, à partir de 0,91 g de n.butylthio-2 phénol, on obtient avec un rendement de 74 % la n.butylthio-3 hydroxy-4 acétophénone.

## Revendications

1. Procédé de préparation d'alkylthio-2-phénols par réaction d'un phénol comportant au moins un atome d'hydrogène en ortho du groupe hydroxyle avec un disulfure de dialkyle linéaire, caractérisé en ce que l'on effectue la réaction en présence de chlorure d'aluminium au sein d'un solvant du type alkyl-benzène choisi parmi le toluène, l'éthylbenzène et le cumène, et en utilisant le chlorure d'aluminium à raison de 1 à 10 moles par mole de phénol.

2. Procédé selon la revendication 1, dans lequel en utilise 1 à 3 moles de chlorure d'aluminium par mole de phénol.

3. Procédé selon la revendication 1 ou 2, dans lequel on utilise de 0,2 à 4 litres de solvant alkyl-benzène par mole de phénol.

4. Procédé selon la revendication 3, dans lequel l'alkyl-benzène est le toluène.

5. Procédé selon l'une des revendications 1 à 4, dans lequel le rapport molaire disulfure/phénol est compris entre 1 et 10, de préférence entre 1 et 5.

6. Procédé selon l'une des revendications 1 à 5, dans lequel le disulfure de dialkyle est le disulfure de diméthyle.

7. Procédé de préparation d'acyl-4 alkylthio-2 phénols, caractérisé en ce que l'on fait réagir a une température allant de 40 à 100°C un alkylthio-2 phénol préparé à partir du phénol par un procédé selon l'une des revendications 1 à 6 et un complexe BF₃ : 2 RCOOH dans lequel R représente un radical alkyle linéaire contenant de 1 à 12 atomes de carbone ou un radical propène-1 yle, à raison de 10 à 15 moles de complexe par mole d'alkylthio-2 phénol.

8. Procédé selon la revendication 7, dans lequel R est un radical méthyle.

9. Procédé selon la revendication 7 ou 8, dans lequel l'alkylthio-2 phénol est le méthylthio-2 phénol.

10. Procédé selon l'une des revendications 7 à 9, dans lequel on utilise environ 12 moles de complexe par mole d'alkylthio-2 phénol.

11. Procédé selon l'une des revendications 7 à 10, dans lequel on opère à une température comprise entre environ 60 et 80°C.

## Claims

1. Process for the preparation of 2-(alkylthio)-phenols by reaction of a phenol containing at least one hydrogen atom ortho to the hydroxyl group with a linear dialkyl disulphide, characterised in that the reaction is carried out in the presence of aluminium chloride in a solvent of the alkylbenzene type chosen from toluene, ethylbenzene and cumene, and employing aluminium chloride in a proportion of 1 to 10 moles per mole of phenol.

2. Process according to Claim 1, in which 1 to 3 moles of aluminium chloride are employed per mole of phenol.

3. Process according to Claim 1 or 2, in which from 0.2 to 4 litres of alkylbenzene solvent are employed per mole of phenol.

4. Process according to Claim 3, in which the alkylbenzene is toluene.

5. Process according to one of Claims 1 to 4, in which the molar ratio disulphide/phenol is between 1 and 10, preferably between 1 and 5.

6. Process according to one of Claims 1 to 5, in which the dialkyl disulphide is dimethyl disulphide.

7. Process for the preparation of 4-acyl-2(alkylthio)phenols, characterised in that a 2-(alkylthio)phenol prepared from phenol by a process according to one of Claims 1 to 6 is reacted at a temperature ranging from 40 to 100°C with a BF₃:2RCOOH complex in which R denotes a linear alkyl radical containing from 1 to 12 carbon atoms or a 1-propenyl radical, in a proportion of 10 to 15 moles of complex per mole of 2-(alkylthio)phenol.

8. Process according to Claim 7, in which R is a methyl radical.

9. Process according to Claim 7 or 8, in which the 2(alkylthio)phenol is 2-(methylthio)phenol.

10. Process according to one of Claims 7 to 9, in which approximately 12 moles of complex are employed per mole of 2-(alkylthio)phenol.

11. Process according to one of Claims 7 to 10, in which the operation is conducted at a temperature of between approximately 60 and 80°C.

## Patentansprüche

1. Verfahren zur Herstellung von Alkylthio-2-phenolen durch Umsetzung eines Phenols, das wenigstens ein Wasserstoffatom in ortho-Position zur Hydroxylgruppe enthält, mit einem linearen Dialkyldisulfid, dadurch gekennzeichnet, daß man die Reaktion in Gegenwart von Aluminiumchlorid in einem Lösungsmittel vom Typ eines Alkylbenzols, ausgewählt aus dem Toluol, dem Ethylbenzol und dem Cumol, durchführt und das Aluminiumchlorid in einer Menge von 1 bis 10 Mol pro Mol Phenol einsetzt.

2. Verfahren nach Anspruch 1, wobei man 1 bis 3 Mol Aluminiumchlorid pro Mol Phenol verwendet.

3. Verfahren nach Anspruch 1 oder 2, wobei man 0,2 bis 4 Liter Lösungsmittel vom Typ eines Alkylbenzols pro Mol Phenol benutzt.

4. Verfahren nach Anspruch 3, wobei das Alkylbenzol Toluol ist.

5. Verfahren nach einem der Ansprüche 1 bis 4, wobei das Molverhältnis Disulfid/Phenol zwischen 1 und 10 liegt und bevorzugt zwischen 1 und 5.

6. Verfahren nach einem der Ansprüche 1 bis 5, wobei das Dialkyldisulfid das Dimethyldisulfid ist.

7. Verfahren zur Herstellung von Acyl-4-alkylthio-2-phenolen, dadurch gekennzeichnet, daß man ein Alkylthio-2-phenol, das, ausgehend vom Phenol, nach einem Verfahren gemäß einem der Ansprüche 1 bis 6 hergestellt wird und eine Komplexverbindung BF₃ : 2RCOOH, wobei R eine lineare Alkylgruppe mit 1 bis 12 Kohlenstoffatomen oder eine 1-Propenylgruppe ist, bei einer Temperatur zwischen 40 und 100°C umsetzt, wobei 10 bis 15 Mol der Komplexverbindung pro Mol Alkylthio-2-phenol eingesetzt werden.

8. Verfahren nach Anspruch 7, wobei R eine Methylgruppe ist.

9. Verfahren nach Anspruch 7 oder 8, wobei das Alkylthio-2-phenol das Methylthio-2-phenol ist.

10. Verfahren nach einem der Ansprüche 7 bis 9, wobei man ungefähr 12 Mol der Komplexverbindung pro Mol Alkylthio-2-phenol einsetzt.

11. Verfahren nach einem der Ansprüche 7 bis 10, wobei man eine Reaktionstemperatur von ungefähr 60 bis 80°C einstellt.
